# EUROPEAN PATENT APPLICATION

(11) **EP 1 053 974 A1**
(43) Date of publication of application: **22.11.2000**
(21) Application number: 99303812.4
(22) Date of filing: 17.05.1999
(51) Int. Cl.: C01G 9/04, C07B 61/00, C07C 29/56, C07C 29/17, C07C 35/12

(54) **Reactions using lewis acids**

(71) Applicant: QUEST INTERNATIONAL B.V., 1411 GP Naarden (NL)
(72) Inventor: The designation of the inventor has not yet been filed
(74) Representative: Matthews, Heather Clare

(57) **Abstract**

In a method of performing a chemical reaction using a Lewis acid, after reaction the Lewis acid is extracted from the reaction product in the form of an aqueous solution, followed by drying of the aqueous solution of the Lewis acid. The dried Lewis acid can be reused in a further similar reaction. The method can be repeated several times, using the same batch of Lewis acid, without the activity and specificity of the Lewis acid being significantly affected. The invention has been used to good effect in the preparation of isopulegol from citronellal using zinc bromide as a Lewis acid catalyst for the cyclisation reaction. The invention can thus provide an environmentally acceptable and efficient process for the production of isopulegol from citronellal, in both racemic and enantiomerically pure forms.

## Description

### Field of the Invention

This invention relates to reactions using Lewis acids and concerns a method of performing a chemical reaction involving a Lewis acid, and products resulting from such reaction.

### Background to the Invention

Lewis acids in the form of metal salts, eg zinc bromide, aluminium chloride etc, are widely used in organic reactions, often on a manufacturing scale, with the Lewis acid commonly functioning as a catalyst, eg in ene-reactions, Diels-Alder reactions and Friedel-Crafts reactions.

Practical difficulties may arise when using Lewis acids in that the Lewis acids may be difficult to recover and reuse. Further, in some cases it may be desirable to use anhydrous forms of Lewis acids for best reactivity and selectivity.

The present invention is based on a novel approach to reactions using a Lewis acid that can overcome such practical difficulties.

### Summary of the Invention

In its broadest aspect the present invention provides a method of performing a chemical reaction involving a Lewis acid, comprising carrying out the reaction involving the Lewis acid; extracting from the reaction products the Lewis acid in the form of an aqueous solution; and drying the aqueous solution of the Lewis acid.

The dried Lewis acid can be reused in a further reaction. It is found that the method of the invention can be repeated several times, using the same batch of Lewis acid, without the activity and selectivity of the Lewis acid being significantly affected. The invention can therefore overcome the problems noted above, enabling quantitative recovery of a Lewis acid and hence enabling repeated reuse of the Lewis acid with consequent reduction in cost of raw materials and avoidance of the cost of disposal of used Lewis acid.

The Lewis acid may be, for example, zinc bromide, zinc chloride, zinc iodide, other metal salts, or mixtures thereof.

The invention is applicable to a range of reactions using Lewis acids, including ene-reactions, Diels-Alder reactions and Friedel-Crafts reactions.

The Lewis acid is conveniently extracted from the reaction products by adding water, preferably deionised water, and separating the aqueous fraction typically from an organic solvent fraction containing other products.

Drying of the aqueous solution of the Lewis acid may be performed using techniques including vacuum dehydration; chemical drying eg with dried molecular sieve material, anhydrous magnesium sulphate or sodium sulphate; physical drying techniques, for instance pervaporisation with selective membrane technology; azeotropic distillation; or a mixture of such techniques.

Azeotropic distillation may be performed using any suitable solvent that gives an azeotrope with water, is inert to the Lewis acid and preferably is suitable for the reaction. Suitable solvents include cyclohexane, benzene, xylene and toluene.

After azeotropic drying, the dried Lewis acid in the solvent is ready for reuse in a reaction and it is simply necessary to add further reagents. The Lewis acid can be recycled and reused in this way several times.

The method of the invention will generally be preceded by an initial step of drying the Lewis acid so that it is in a suitable state to take part in the reaction, eg to act as a catalyst in the reaction. Drying may be performed by the techniques described above, and is preferably performed by azeotropic distillation eg with toluene of an aqueous solution of the Lewis acid. Such initial drying has the beneficial consequence that it is not necessary to use the Lewis acid reagent in anhydrous form. Some Lewis acids, eg zinc bromide, are expensive and difficult to maintain in anhydrous condition, being hygroscopic. It is thus beneficial not to have to use the reagent in anhydrous form but instead to be able to use an aqueous solution of Lewis acid, which is a cheaper starting material that does not require special storage and handling.

In a preferred aspect the invention provides a method of performing a chemical reaction involving a Lewis acid, comprising the steps of:
1) drying an aqueous solution of the Lewis acid;
2) using the dried Lewis acid in the chemical reaction;
3) adding water to the reaction products of step 2) and separating an aqueous solution of the Lewis acid; and
4) repeating steps 1) to 3).

Steps 1) to 3) can be repeated several times, thus reusing the same Lewis acid in several reactions, without significantly affecting the activity and specificity of the Lewis acid in the reaction.

The invention has been used to good effect in the preparation of isopulegol from citronellal. Isopulegol and citronellal both exist in two enantiomeric forms. Mixtures of enantiomers of each of these materials are nevertheless referred to in the singular, eg as isopulegol, so that references to isopulegol may refer to one or other enantiomeric form or a mixture of enantiomers. Similar considerations apply to citronellal.

Isopulegol is a known fragrance material, with its most important use being as a precursor for menthol in a known hydrogenation reaction. L-menthol (or (-)-menthol) is particularly favoured and is widely used, eg in dental flavours. It is known to make isopulegol by cyclisation of citronellal using a Lewis acid catalyst. Powdered anhydrous zinc bromide has been found to be one of the most selective catalysts in the cyclisation reaction, as described in Nakatani et al, Synthesis 1978, 147. In this known process zinc bromide is required in stoichiometric amounts and is effectively consumed in the process, and moreover can present waste disposal problems, as zinc bromide is a marine toxin. Zinc bromide is also hygroscopic and wet zinc bromide is much less reactive than the anhydrous form. Hence precautions are required in the storage and usage of zinc bromide to avoid moisture.

Thus, in a preferred aspect of the invention there is provided a method of preparing isopulegol from citronellal, comprising reacting citronellal in the presence of a Lewis acid catalyst to cause cyclisation of citronellal to produce isopulegol; extracting from the reaction products the Lewis acid in the form of an aqueous solution; and drying the aqueous solution of the Lewis acid.

As noted above isopulegol exists in two enantiomeric forms. For use as a precursor for production of menthol, it is desirable to have 1-isopulegol (or (-)-isopulegol) as this produces 1-menthol which is generally the most preferred form of menthol, as noted above. When using d-citronellal (or (+)-citronellal) as the starting material, the invention shows good selectivity for 1-isopulegol in preference to other isomers, so the reaction takes place without loss of optical purity. The reaction of d-citronellal to produce 1-isopulegol and subsequent conversion into 1-menthol can be represented as follows:

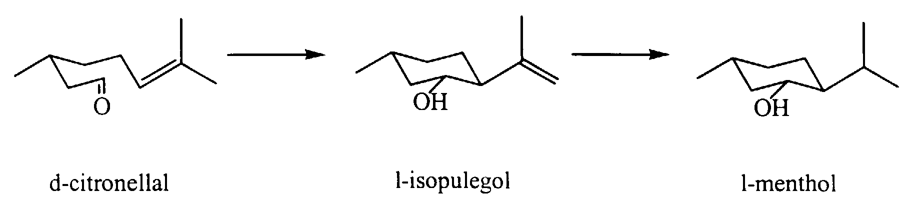

Similarly, by starting with a racemic mixture of citronellal, ie equal amounts of the d-and 1- forms, a racemic mixture of isopulegol and then menthol can be produced. Thus, the optical purity of the materials is maintained through the process. As well as maintaining optical purity, the invention can produce isopulegol in good yields.

The dried Lewis acid can be reused in the cyclisation of a further batch of citronellal to isopulegol, without loss of reactivity and selectivity. The invention can therefore overcome the problems noted above associated with the prior art, enabling quantitative recovery of Lewis acid and hence enabling repeated reuse of Lewis acid with consequent reduction in cost of raw materials and avoidance of the cost of disposal of used zinc bromide.

The Lewis acid may be, for example, zinc bromide, zinc chloride, zinc iodide, other metal salts or mixtures thereof. Zinc bromide generally gives the best yield and selectivity and so is favoured in the conversion of citronellal to isopulegol.

The cyclisation reaction is carried out in a suitable organic solvent, such as cyclohexane, benzene, xylene, toluene or mixtures thereof. The currently preferred solvent is toluene as it gives good reaction rates and selectivity. Toluene is also a cheap, safe solvent and so is well suited to industrial use.

The cyclisation reaction is conveniently carried out at a temperature in the range -15°C to 30°C, with a temperature of about 0°C giving good compromise results in terms of yield and selectivity.

The Lewis acid is conveniently extracted from the reaction products by adding water, preferably deionised water, and separating the aqueous fraction from the organic solvent fraction containing isopulegol.

Drying of the aqueous solution of the Lewis acid may be performed using techniques including vacuum dehydration; chemical drying eg with dried molecular sieve material, anhydrous magnesium sulphate or sodium sulphate; physical drying techniques, for instance pervaporisation with selective membrane technology; azeotropic distillation; or a mixture of such techniques. Of the techniques tested, azeotropic distillation is currently favoured as the resulting dried Lewis acid is found to give best product yields and selectivity. It may be convenient to precede azeotropic distillation by optional vacuum dehydration.

Azeotropic distillation may be performed using any suitable solvent that gives an azeotrope with water, is inert to the Lewis acid and preferably is suitable for the cyclisation reaction. Suitable solvents include those mentioned above, ie cyclohexane, benzene, xylene and toluene, with toluene currently being favoured.

After azeotropic drying, the dried Lewis acid in the solvent is ready for reuse in the cyclisation reaction and it is simply necessary to add a further batch of citronellal. The Lewis acid can be recycled and reused in this way several times.

The preferred method of the invention will generally be preceded by an initial step of drying the Lewis acid so that it is in a suitable state to act as a catalyst in the cyclisation reaction. Drying may be performed by the techniques described above, and is preferably performed by azeotropic distillation with toluene of an aqueous solution of the Lewis acid. Such initial drying has the beneficial consequence that it is not necessary to use as a reagent anhydrous Lewis acid, eg zinc bromide, which is expensive and difficult to maintain in anhydrous condition as it is a hygroscopic material; instead an aqueous solution of Lewis acid can be used as a reagent, which is a cheaper starting material that does not require special storage and handling.

In a further preferred aspect the invention thus provides a method of preparing isopulegol from citronellal, comprising the steps of:
1) drying an aqueous solution of zinc bromide by azeotropic distillation using toluene;
2) using the dried zinc bromide as a catalyst for cyclisation of citronellal to produce isopulegol;
3) adding water to the reaction products of step 2) and separating an aqueous solution of zinc bromide from the toluene solution of isopulegol; and
4) repeating steps 1) to 3).

Steps 1) to 3) can be repeated several times, thus reusing the same zinc bromide on several batches of citronellal, without significantly affecting the activity and specificity of the zinc bromide in the cyclisation reaction. The method can give good yields of isopulegol of desired optical form even after repeated re-use of the zinc bromide.

The invention can thus provide an environmentally acceptable and efficient process for the production of isopulegol from citronellal, in both racemic and enantiomerically pure forms.

The invention includes within its scope isopulegol prepared by the method of the invention.

The isopulegol can be converted into menthol, eg by hydrogenation.

The invention also covers use of the isopulegol so prepared for the preparation of menthol, and the resulting menthol.

As noted above, the invention enables conversion of d-citronellal to 1-isopulegol and then 1-menthol, while a racemic starting material produces racemic products.

The invention will be further described, by way of illustration, in the following Examples.

### Example 1 - Drying of zinc bromide.

In a 250ml 3-neck flask, equipped with a Dean & Stark (D&S) trap. 80ml toluene and 50.21 gram 80%(w/w) aqueous zinc bromide solution were added. The mixture was heated up to boiling and water was trapped and separated into lower layer as the distillation proceeded. About 8.8ml of water was trapped and there was no increase in the amount of water after refluxing for 8 hours. A white, slightly pink, suspension resulted. The suspension was then cooled to 4°C.

Racemic citronellal (68.80g, 0.45 mol) was added to the suspension over 3 hours by pump at 0.4g/min. at 2-5°C and stirred vigorously. The citronellal had purity 91.81% rpa with 4.35% isopulegol and 2.22% pulegol, ORD=+2.0. The resulting mixture was stirred for another 1.5 hours. According to GC, the reaction was completed by this stage. The reaction mixture was then quenched with deionised (DI) water (3x100ml). The organic layer was then washed with brine (lx50ml). The yield of isopulegol was found to be 87.14% by GC analysis.

The zinc content of the aqueous solution was determined by atomic absorption analysis and it was found that zinc was totally recovered in the aqueous extraction.

The recovered zinc bromide was dried and used in a further cyclisation reaction under the same conditions. Thus, 200ml toluene was added to the zinc bromide solution, and the mixture was refluxed with a D&S trap for 8 hours. Citronellal was pumped in to the suspension at 0.4g/min. for 2 hours 15 min at 2-5°C. 53.95g citronellal was added and the solution was stirred for 3 more hours. According to GC the reaction was complete by this stage. The reaction mixture was then washed with deionised water (2x50ml). The yield of isopulegol was found to be 85.85% by GC analysis. Moreover, the diastereoselectivity of the two cycles were similar.

### Example 2 - Repeated recycling of zinc bromide

Experiments similar to those of Example 1 using azeotropic drying of toluene were carried out on a larger scale using reagents from the same source. The experiments were carried out using a 10 litre jacketed reactor with a Dean & Stark trap, condenser, mechanical stirrer and thermocouple.
- GC Method:: GC: HP6890 series
Column: Model No. HP 19091J-412
HP-5 5% PhenylMethylsilxane Capillary
30.0mX320µmX0.25µm nominal
- Temp Program:: 70°C, hold 1 minutes, ramp 3°C/min., 280°C

### a) First Cycle, Dehydration of Zinc Bromide Solution.

In a 101 jacketed reactor, equipped with a D & S trap and thermocouple, 2.51 toluene was added and the reactor was heated up to 126°C, at which point toluene was refluxed at a reasonable rate. To the refluxing toluene, with an addition funnel, the zinc bromide solution (350.40g zinc bromide in 600ml water) was added slowly over 2 hours with vigorous stirring (∼80rpm). The reflux continued for another 8 hours. 600ml water was removed and no more water was trapped. A pinkish white suspension was resulted.

### b) First Cycle, Cyclisation of Citronellal

To the suspension resulting from step (a) cooled to 0°C, stirred vigorously (∼80rpm), racemic citronellal was pumped in at 4.76g/min. 600.24g citronellal was added after 2 hours and 6 min. The solution (most of the zinc bromide was dissolved at this point) was further stirred at +1-0°C for 1.5 hours. According to GC and TLC, the reaction was completed by this stage. To the reaction mixture, 600ml DI water was added with stirring. The aqueous phase was separated and removed. The organic phase was washed with another 600ml DI water and separated. The yield of isopulegol was found to be 88.29% by GC analysis.

The pH of the zinc bromide solution = 5.47

### c) Second Cycle, Dehydration of Zinc Bromide Solution

In a 101 jacketed reactor, equipped with a D&S trap and thermocouple, 2.51 toluene was added and the reactor was heated up to 126°C, at which point toluene was refluxed at a reasonable rate. To the refluxing toluene, with an addition funnel, the zinc bromide solution and the combined aqueous phase from step (b) was added slowly over 3 hours with vigorous stirring (∼80rpm). The reflux continued for 7.5 hours. A suspension of zinc bromide in toluene was resulted.

### d) Second Cycle, Cyclisation of Citronellal.

To the suspension resulting from step (c) cooled to 0°C, stirred vigorously (∼80rpm), racemic citronellal was pumped in at 4.76g/min. 600.16g citronellal was added after 2 hours and 6 min. The solution (most of the zinc bromide was dissolved at this point) was further stirred at +1-0°C for 1.5 hours. According to GC and TLC, the reaction was completed by this stage. To the reaction mixture, 600ml DI water was added with stirring. The aqueous phase was separated and removed. The organic phase was washed with another 600ml DI water and separated. The yield of isopulegol was found to be 87.90% by GC analysis with similar disastereoselectivity compare with the first cycle.

The pH of the zinc bromide solution = 5.56

### e) Third Cycle, Dehydration of Zinc Bromide Solution.

To the 1.2 litre zinc bromide solution from step (d), procedure (c) was applied. A pinkish suspension of zinc bromide in toluene was resulted.

### f) Third Cycle, Cyclisation of Citronellal.

To the suspension resulting from step (e) cooled to 0°C, stirred vigorously (∼80rpm), citronellal was pumped in at 4.76g/min. 694.61g citronellal was added after 2 hours and 40 min. The solution (most of the zinc bromide was dissolved at this point) was further stirred at +2-0°C for 1.5 hours. According to GC and TLC, the reaction was completed. by this stage. To the reaction mixture, 600ml DI water was added with stirring. The aqueous phase was separated and removed. The organic phase was washed with another 600ml DI water and separated. The yield of isopulegol was found to be 82.26% by GC analysis with similar disastereoselectivity compare with the first cycle.

The pH of the zinc bromide solution = 5.36

### g) Fourth Cycle, Dehydration of Zinc Bromide solution

To the 1.21 zinc bromide solution from step (f), procedure (c) was applied. A suspension of zinc bromide in toluene was resulted.

### h) Fourth Cycle, Cyclisation of Citronellal.

To the suspension resulting from step (g) cooled to 0°C, stirred vigorously (∼80rpm), racemic citronellal was pumped in at 4.76g/min. 600.10g citronellal was added after 2 hours and 14 min. The solution (most of the zinc bromide was dissolved at this point) was further stirred at +2-0°C for 1.5 hours. According to GC and TLC, the reaction was completed by this stage. To the reaction mixture, 600ml DI water was added with stirring. The aqueous phase was separated and removed. The organic phase was washed with another 600ml DI water and separated. The yield of isopulegol was found to be 86.22% by GC analysis with similar disastereoselectivity compared with the first cycle.

The pH of the zinc bromide solution = 5.08

The pH of the aqueous zinc bromide after each cycle was measured to see if substantial hydrolysis occurred. There was a very slight drop in pH after 4 cycles.

This Example shows that the selectivity and reactivity of the zinc bromide were maintained at similar levels, as the zinc bromide was recycled three times. From the pH study, no significant hydrolysis took place when zinc bromide was repeatedly recycled.

Similar results have been obtained with manufacturing scale reactions.

### Example 3 - Cyclisation of d-citronellal to 1-isopulegol

Experiments were carried out using commercially available d enriched citronellal, having a d/l enantiomeric ratio of about 90/10. Isopulegol having a l/d enantiomeric ratio of about 90/10 was produced, showing that optical purity is maintained when using as catalyst in the cyclisation reaction zinc bromide dried by azeotropic distillation of an aqueous solution.
- Materials:: Citronellal: 90% min.
refractive Index: 1.448
optical rotation, [α]D: + 11.2 ([α]_{D} of d-citronellal= + 10.6, reported in Dictionary of Organic Compounds, 4^{th} Ed. Vol. 2 pp. 716)
d/l enantiomeric ratio= 90.60/9.40 measured by chiral
GC (condition specified below)
- Zinc Bromide:: 98% min.
Chiral GC condition:

### CITRONELLAL

Machine: Carlo Erba HRGC5300 with split injector and FID Column: Diacetyl tert-butyldimenthylsilyl beta cyclodextrin Dimensions: 25m*0.25mm*0.15µm
Linear velocity: 16.69cm/sec
Temperature Prog: 80° 1°/min 150°(10min) Flow rate: 0.473ml/min
Split Flow: Ratio 200:1
Concentration: 0.3%
Retention time: t=23.544 min
t=24.085 min
Resolution achieved: separation Rs=1.29

### ISO-PULEGOL

Machine: HP 5890 GC with split injector and FID
Column: CP-Cyclodextrin-B-236-M-19
Dimensions: 48m∗0.25mm∗0.25µm
Linear velocity: 23.8cm/sec
Temperature Prog: 70° 3°/min 200° Flow rate: 1.14ml/min
Split Flow: 130:1
Concentration: 0.1%
Name of the sample (-)iso-pulegol + racemic isopulegol Retention time: t=∼21.62 (5%) (+)iso-pulegol t=21.78 (81%) (-)iso-pulegol
NOTE:- (-)iso-pulegol co-elutes with one enantiomer of iso-iso-pulegol

### Procedure

To 200ml toluene in a 1 litre 3-neck flask, equipped with water-cooled condenser, Dean & Stark trap, mechanical stirrer, thermometer and addition funnel, heating under gentle reflux, a solution of zinc bromide (made by dissolving 29.14g zinc bromide in 30ml deionised water) was added over 30 minutes. The heating was continued for 3 hours; no more water was collected in the trap. A pale pink suspension of zinc bromide in toluene resulted. The suspension was cooled with an ice bath to +3°C. 44.68g of citronellal was added dropwise over 2 hours and the temperature was kept at 0-3°C. The mixture was then further stirred for another 2 hours. 60ml deionised water was then added to the reaction mixture, stirred vigorously for 1 minute and the aqueous layer was removed and the organic layer was washed with another 60ml portion of deionised water. A toluene solution of isopulegol was obtained. The ratio of the enantiomers of isopulegol was then measured by chiral GC analysis under the condition as detailed above. The ratio of isopulegol l:d was found to be 90.90:9.10.

### Example 4 - Hydrogenation of isopulegol to menthol

Crude isopulegol produced as described in Example 1, using zinc bromide dried by azeotropic distillation (containing 73.31% isopulegol) was hydrogenated after removal of all the toluene.

To the isopulegol (257.89g, 1.227mol) in 1 litre Buchi flask, 5% Pd/C (4.84g, 2.28 mmol metal) was added. The suspension was purged with nitrogen and pressurised with hydrogen to 5 bars. The suspension was then stirred at 1200 rpm at room temperature and hydrogen pressure was maintained at 2-5 bars. The reaction was followed by GC and was shown to be completed in 2 hours. The catalyst was then filtered off using a plug of Celite (Celite is a Trade Mark) and the filtered catalyst was washed with hexanes (3x30ml). (Celite is a diatomaceous earth, about 95% SiO₂). After removal of solvent, 260.8g crude product was obtained. Isopulegol was found to be quantitatively hydrogenated.

## Claims

1. A method of performing a chemical reaction involving a Lewis acid, comprising carrying out the reaction involving the Lewis acid; extracting from the reaction products the Lewis acid in the form of an aqueous solution; and drying the aqueous solution of the Lewis acid.

2. A method according to claim 1, wherein the Lewis acid is extracted from the reaction products by adding water, and separating the aqueous fraction from an organic solvent fraction containing other reaction products.

3. A method according to claim 1 or 2, wherein drying of the aqueous solution of the Lewis acid is performed by azeotropic distillation.

4. A method according to claim 1, 2 or 3, wherein the dried Lewis acid is used in a further reaction.

5. A method according to any one of the preceding claims, preceded by an initial step of drying the Lewis acid.

6. A method of performing a chemical reaction involving a Lewis acid, comprising the steps of:
1) drying an aqueous solution of Lewis acid;
2) using the dried Lewis acid in the chemical reaction;
3) adding water to the reaction products of step 2) and separating an aqueous solution of the Lewis acid; and
4) repeating steps 1) to 3).

7. A method of preparing isopulegol from citronellal, comprising reacting citronellal in the presence of a Lewis acid catalyst to cause cyclisation of citronellal to produce isopulegol; extracting from the reaction products the Lewis acid in the form of an aqueous solution; and drying the aqueous solution of the Lewis acid.

8. A method according to claim 7, wherein the Lewis acid is zinc bromide.

9. A method according to claim 7 or 8, wherein the cyclisation reaction is carried out in toluene.

10. A method according to claim 7, 8 or 9, wherein the cyclisation reaction is carried out at a temperature in the range -15°C to 30°C, preferably about 0°C.

11. A method according to any one of claims 7 to 10, wherein the Lewis acid is extracted from the reaction products by adding water, and separating the aqueous fraction from an organic solvent fraction containing isopulegol.

12. A method according to any one of claims 7 to 11, wherein drying of the aqueous solution of the Lewis acid is performed by azeotropic distillation.

13. A method according to claim 12, wherein the azeotropic distillation is performed using toluene.

14. A method according to claim 12 or 13, wherein the azeotropic distillation is preceded by vacuum dehydration.

15. A method according to any one of claims 7 to 14, wherein the dried Lewis acid is used as catalyst in a further cyclisation reaction of citronellal to produce isopulegol.

16. A method according to any one of claims 7 to 15, preceded by an initial step of drying the Lewis acid.

17. A method of preparing isopulegol from citronellal, comprising the steps of:
1) drying an aqueous solution of zinc bromide by azeotropic distillation using toluene;
2) using the dried zinc bromide as a catalyst for cyclisation of citronellal to produce isopulegol;
3) adding water to the reaction products of step 2) and separating an aqueous solution of zinc bromide; and
4) repeating steps 1) to 3).

18. A method according to any one of claims 7 to 17, wherein 1-isopulegol is prepared from d-citronellal, without change in optical purity.

19. A method according to any one of claims 7 to 18, further comprising converting the resulting isopulegol to menthol.

20. Isopulegol prepared by the method of any of claims 7 to 19.

21. Menthol prepared by the method of claim 20.
